# EUROPEAN PATENT SPECIFICATION

(11) **EP 4 436 703 B1**
(45) Date of publication and mention of the grant of the patent: **16.07.2025**
(21) Application number: 23714609.7
(22) Date of filing: 28.03.2023
(51) Int. Cl.: B01J 3/00, B01J 19/02, F16K 25/00, F16K 27/00

(54) **UREA PLANT WITH VALVE; UREA PRODUCTION PROCESS; USE AND METHOD**
HARNSTOFFANLAGE MIT VENTIL, VERFAHREN ZUR HERSTELLUNG VON HARNSTOFF, VERWENDUNG UND VERFAHREN
INSTALLATION DE PRODUCTION D'URÉE DOTÉE D'UNE VALVE, PROCÉDÉ DE PRODUCTION D'URÉE, UTILISATION ET PROCÉDÉ

(30) Priority: 28.03.2022 EP 22164875
(43) Date of publication of application: 02.10.2024
(73) Proprietor: Stamicarbon B.V., 6135 KW Sittard (NL)
(72) Inventor: JORISSEN, Paul Maria Gerardus Clemens Petrus Hubertus, 6135 KW Sittard (NL); SCHEERDER, Alexander Aleida Antonius, 6135 KW Sittard (NL); OFEI, Kirk Anguah, 6135 KW Sittard (NL); BRUN, Alessandro, 8605 Kapfenberg (AT); SIMML, Michael, 8605 Kapfenberg (AT)
(74) Representative: V.O.
(86) International application number: PCT/NL2023/050157
(87) International publication number: WO 2023/191620

(56) References cited:
- EP-A1- 3 502 294
- WO-A1-2008/000740
- WO-A1-2015/099530
- WO-A1-2021/006729
- CN-U- 210 318 513
- DE-A1- 2 248 079
- GB-A- 1 325 546
- GB-A- 2 073 751
- KR-A- 20180 043 651

## Description

### Field

The invention pertains, in some embodiments, to a urea production plant comprising a valve.

### Introduction

Example urea production plants are illustrated in Ullmann's Encyclopaedia of Industrial Chemistry, Chapter Urea, 2010.

GB2073751 describes a valve with parts made with different materials, according to the fluid to be handled in a urea synthesizing plant.

In urea plants the used equipment must have a proper resistance against the process media. Materials such as duplex stainless steel are used for obtaining a proper lifetime and resistance against corrosion due to the aggressive properties of the media involved in the urea production process. In particular, the carbamate-containing process streams are highly corrosive, more in particular at the relatively high temperatures occurring in the high pressure synthesis section.

It is generally desired to have better valves for urea plants, in particular with better corrosion resistance, and good mechanical properties as well as relatively low capital expenditure. This desire is particularly important for high pressure valves, more in particular for high pressure control valves.

Valves in urea plants are typically made of urea grade steel, for example duplex stainless steel.

The brochure "BHDT High Pressure Products for a Safe and Reliable Urea Industry", http://www.ureaknowhow.com , discusses various valves for urea plants.

An example method of manufacturing single body duplex stainless steel units is providing a sufficiently large block of the duplex stainless steel followed by shaping, such as forging, the material and additional heat treatment to effect a change in micro structure in the solid state. The heat treatment can be challenging for large sizes of the equipment due to possibly inhomogeneous heating and cooling.

At the end of the lifetime of the valve, or at least at the end of the lifetime of the valve body, conventionally the entire valve is replaced.

There remains a desire for better valves for urea plants, and for urea plants having better valves, and urea production processes using such valves and for uses of such valves.

### Summary

The invention pertains in a first aspect to urea production plant, comprising a valve as defined in claim 1.

The invention also provides a use of the valve in a carbamate environment; said use comprising exposing the inner body part to a liquid comprising carbamate and/or to a gas stream comprising CO₂ and NH₃.

The invention also pertains to a urea production process comprising forming urea from NH₃ and CO₂ in a urea production plant according to the invention.

The invention also pertains to a method of modifying an existing urea production plant comprising a first valve, by replacing the first valve by a valve as described herein.

### Brief description of the drawings

Figure 1 schematically illustrates an example valve for use in the invention.
Figures 2-6 illustrate different views of an example valve for use in the invention.
Fig. 7 and 8 illustrate example urea production plants according to the invention.

Any embodiments illustrated in the figures are examples only and do not limit the invention.

### Detailed description

The inner body part of valve used in the invention has a relatively small size, in particular a smaller wall thickness compared to a single body valve with an equivalent flow rate, and is made from duplex stainless steel with proper mechanical as well as corrosion resistance properties over the full thickness. In particular the correct microstructure of duplex stainless steel can be more easily obtained. In particular the maximum depth of the inner body part, wherein the depth is the minimum distance from an point inside the inner body part to surface of the inner body part, is smaller compared to a reference valve body forged of a single metal piece, which is advantageous for the final heat treatment of the inner body part, especially for duplex stainless steel inner body parts.

The outer body part of the valve is not in direct contact with carbamate-containing liquid in operation and therefore can be made from a less corrosion resistant material. This advantageously reduces capital expenditure.

The use of the inner body part and the outer body part that are fit together and/or mechanically joined to each other, provides the advantage that relatively complex shapes can be achieved for the chamber which receives fluid in operation. This provides an advantage e.g. over lining.

A lining process involves applying on the internal equipment surfaces in contact with the media (surfaces to be protected against corrosion) a thin layer of a suitable material, such as PTFE; typically, a polymeric material. The lining process is generally not applicable for valves due to several limitations including the maximum allowable temperature being too close to the operating temperatures in chemical plants such as a high pressure (HP) section of a urea plant. The lining material is a soft thermoplastic material that cannot guarantee a proper lifetime against erosion, corrosion and/or cavitation. A soft thermoplastic material furthermore cannot guarantee a proper lifetime against the high pressure involved (e.g. at least 100 bar).

Cladding with duplex stainless steel by welding is generally not an attractive option for valve bodies due to the complex internal geometry of the valve body. Moreover, unavoidable microporosity of cladding can jeopardize valve functionality.

The inner body part of the valve used in the invention is made of duplex stainless steel.

The invention pertains to a urea production plant (system). The product for example comprises urea ammonium nitrate, urea ammonium sulphate, for example urea-containing fertilizers. The product may also be, e.g., diesel exhaust fluid (DEF) or a urea feed stream for a melamine plant, or urea for other uses, or combinations thereof.

The plant comprises a high pressure (HP) synthesis section for urea synthesis. The HP synthesis section comprises a reaction zone and for example also comprises a high pressure (HP) stripper and a high pressure (HP) carbamate condensation zone. The HP stripper is for instance of the CO₂ stripping type, or for instance of the self-stripping or NH₃ stripping type. In the HP synthesis section, NH₃ and CO₂ feeds are reacted to from urea. The reaction zone is for instance provided, at least in part, by a vertical urea reactor with trays, having one or more inlets at the bottom and one or more outlets at the top.

The HP stripper is for instance a vertical shell-and-tube heat exchanger with a falling film of urea solution in the tubes. The HP stripper for instance has an inlet for strip gas, e.g. CO₂ gas stream, at the bottom, and an outlet for stripped urea solution at the bottom and an outlet for a gas stream at the top connected to an inlet of the HP carbamate condensation zone.

The HP condensation zone is for instance a shell-and-tube heat exchanger, for instance a vertical or a horizontal shell-and-tube heat exchanger, with for example at least one tube bundle comprising straight or U-shaped tubes. In some embodiments, the reaction zone and the condensation zone are combined in a single vessel, for example in a pool reactor. The HP carbamate condensation zone has a fluid connection to the HP reaction zone, for example the HP carbamate condenser has an outlet connected to an inlet of the reactor.

In an example embodiment, the urea plant is of the CO₂ stripping type and the HP stripper has an inlet for CO₂ at the bottom and preferably stripper tubes made of duplex stainless steel, with preference for the same steel alloys as preferred for the inner body part of the valve. In this embodiment, the HP carbamate condenser is, for example, a horizontal shell-and-tube heat exchanger receiving cooling fluid in the tubes and the gas from the stripper in the shell, and for example with a U-shaped tube bundle.

In example embodiment, the urea plant is of the self-stripping type, and the HP stripper comprises for example bimetallic stripper tubes. In this embodiment, the HP carbamate condenser is for instance of the kettle-type with a U-shaped tube bundle, receiving cooling fluid in the shell and the gas from the stripper in the tubes.

The urea plant comprises a recovery section. Herein, the recovery section refers to a section for purification of the urea synthesis solution from the HP synthesis section to increase the urea content. The urea synthesis solution from the HP synthesis section comprises urea, water, NH₃, and carbamate.

Preferably, the recovery section contains a low pressure (LP) recovery section, and optionally a medium pressure (MP) recovery section. Preferably, the recovery section comprises a low pressure (LP) and/or a medium pressure (MP) decomposer for removing carbamate from the urea solution, and having a gas outlet. Preferably, the gas stream is in part or entirely condensed in a carbamate condenser and the resulting carbamate solution is recycled to the HP synthesis section. The urea plant is for instance of the total recycle design. Alternatively, the urea plant is of the partial recycle design.

The urea plant is for instance and without limitation of the CO₂ stripping type, the self-stripping type, or the NH₃ stripping type, with as non-limiting examples, the Stamicarbon CO₂ stripping design, the Saipem self-stripping design, and the Toyo CO₂ stripping design.

Optionally, the urea production plant is a part of a melamine production plant.

The valve is provided in the HP synthesis section. Optionally, the valve is also provided in an MP recovery section, or possibly in an LP recovery section. The inventive valve is comprised in the HP synthesis section.

The valve preferably receives a fluid stream, comprising or consisting of a liquid, of at least 100 bar, more preferably at least 140 bar, e.g. less than 200 bar. The fluid stream for example is a urea solution, for instance a stripped urea solution, comprising at least 10 wt.% or at least 15 wt.% ammonium carbamate. The fluid stream, e.g. a urea solution, has a temperature of e.g. at least 150°C, e.g. in the range 160-220°C.

The valve is for instance, without restriction, designed as a control valve (regulating valve), or an on-off valve, or a manually operated valve.

The valve can be used for fluids, such as gases or liquids. For example, but without limitation, the valve is used for urea solution, or for off-gas from the HP synthesis section. The valve can be used, for example, as a control valve, for example as a reactor letdown valve, or as an isolating on-off valve. Off-gas streams from a urea plant comprise NH₃ and CO₂ and cause a risk of condensation corrosion.

The invention also pertains to a process for producing urea comprising reacting NH₃ and CO₂ under urea forming conditions in a urea production plant, wherein the plant comprises at least one valve according to the invention; preferably the plant is a urea production plant according to the invention. Urea forming conditions include e.g. a pressure of above 100 bar, e.g. in the range 100 - 400 bar, and a temperature above 120°C, e.g. in the range 150-250°C.

The process preferably comprises forming ammonium carbamate, and dehydrating ammonium carbamate to provide urea and water.

Preferably, the process is of the CO₂ stripping, the NH₃ stripping, or the self-stripping type; with further preferences the same as for the plant.

All preferences for the valve discussed in connection with the plant apply also for the process. All preferences for the plant, e.g. the type of plant and the type of synthesis section, apply also for the plant in which the process is carried out. All preferences for the process also apply for the plant.

The invention also pertains to the use of a valve according to the invention (or a valve as used in a plant according to the invention) in a carbamate environment; said use comprising exposing the inner body part to a liquid comprising carbamate and/or to a gas stream comprising CO₂ and NH₃. This particularly pertains to a concentrated carbamate solution, such as an ammonium carbamate solution having a concentration of from 15 wt.% to 95 wt.% of ammonium carbamate. Preferably, the inner body part is in direct contact with such a carbamate solution during the use or during the process. All preferences for the valve discussed in connection with the plant and the process apply also for the valve according to the use.

The invention also provides a method of modifying an existing urea production plant comprising a first valve, by replacing the first valve by a valve comprising a valve body comprising an inner body part and an outer body part as described herein. All preferences for the valve discussed in connection with the urea plant apply equally to the method of modifying an existing urea production plant. The method preferably gives an inventive plant comprising the valve. The replaced valve is for instance a high pressure (HP) control valve.

All features, preferences and details of the valve discussed in the following paragraphs apply equally for all aspects of the invention, including the urea production plant, the process, the use and the method of modifying an existing urea production plant.

The valve generally can be used for stopping and starting fluid flow, or varying the amount of fluid flow, or for example controlling the direction of fluid flow, or for example relieving over pressure. The valve can also be referred to as a device for controlling passage of fluid or a device for regulating fluid flow.

The valve body, which may also be referred to as a valve shell, is the primary pressure boundary of the valve. The valve body holds the elements of the valve together. The valve body in operation resists fluid pressure loads from connecting pipes. The valve body receives inlet and outlet piping through joints. The valve body typically contains an opening for assembly of the valve; in particular for assembly of flow regulating internal elements with the valve body.

The valve comprises a first opening and a second opening in the valve body for fluid to enter and exit the valve body. The openings are in particular configured for allowing the fluid to enter and exit the chamber for holding fluid, wherein said chamber is provided in the valve body. The valve hence comprises an inlet and an outlet. The valve may comprise two or more inlets and may comprise two or more outlets.

The valve also comprises a flow-regulating member, i.e. a flow-regulating internal element, for example a regulating stem or a disc. The flow regulating member can move in the chamber between at least two different positions for regulating the fluid flow. The flow regulating member is located in the chamber. The valve typically comprises a stem connecting the flow regulating member to a controlling device, such as an actuator or a handle. The flow regulating member is e.g. a plug and is for instance a pressure retaining part. The flow regulating member is, typically, a movable obstruction inside the valve body that adjustably restricts fluid flow through the valve. The flow regulating member is typically capable of being moved to a position wherein a passageway or a port of the valve is obstructed in order to regulate the fluid flow. The flow regulating member is also known as a disc; the flow regulating member may have various shapes.

The valve for example comprises a valve seat, providing a seating surface for the flow regulating member. The seat can be a surface of the valve body, but is preferably an element of the valve that is separate from the valve body. The valve seat for example provides an interior surface of the valve body which contacts the flow regulating member to form a leak-tight seal in at least one position of the flow regulating member.

The flow regulating member is for example connected by a stem to controlling device such as an actuator. The actuator is e.g. located outside the valve body. The stem for instance transmits motion from a handle or actuator to the flow regulating member. The actuator is for instance a controlling device of the valve and e.g. comprises a motor.

The valve typically comprises a removable piece making assembly of the valve possible in particular assembly of the inner body part in the recess of the outer body part. In some embodiments, this piece is provided by the valve seat and a seat connection piece. The removable piece is jointed to the valve body e.g. with bolts. The removable piece may comprise an opening for fluid flow and may be connected to a pipe.

The valve for example has a valve body size of 4 inch (10.2 cm) or higher, such as 8 inch (20.3 cm) or higher. The valve for example has a nominal bore diameter of 2.75 inch (70 mm) or higher, such as 5.60 inch (142 mm) or higher and/or the valve for example has a seat diameter of 0.39 inch (10 mm) or higher, or 3.94 inch (100 mm) or higher, for instance 6.70 inch (170 mm) or higher.

The valve body comprises the chamber for receiving fluids. In the valve of the present disclosure, the inner body part provides the chamber. The walls of the chamber are provided by the inner body part. In particular, the walls of the chamber are provided only by the inner body part and not by the outer body part. The chamber is comprised in the inner body part.

The valve is for instance, without restriction, an angle type globe valve, a straight type globe valve, a butterfly valve, or a check valve. The angle type globe valve is e.g. a control valve or an on-off valve. The straight type globe valve is e.g. a control valve or an on-off valve. The butterfly valve is e.g. a control valve or an on-off valve.

The valve for instance comprises a movable plug or disc element and a stationary valve seat. The plug typically can be moved in the chamber configured for receiving fluids in the body.

The valve seat provides a stable, uniform and typically replaceable shut off surface. The valve seat is for instance a ring seat.

The valve is for instance, without restriction, designed to withstand at least 50 bar gauge or at least 100 bar gauge pressure, or at least 240 bar gauge, with the high pressure fluid inside.

The valve body comprises the inner body part and the outer body part. The inner body part and outer body part are fit together and/or mechanically joined to each other. The inner body part is for example mechanically held in the outer body part, in particular in a recess of the outer body part. The inner body part is for instance fitted in a recess of the outer body part. In this way, the inner and outer body part can be fit together. For example, an interference fit or a friction fit is used. The inner body part and outer body part are for example mechanically attached to each other. The inner body part is typically mechanically held in place in the outer body part, for example by a seat connection piece. The foregoing provides illustrative examples of the inner and outer body parts being mechanically joined to each other. Hence, the inner body part and outer body part are separate and disjoint pieces. An interference fit and friction fit are suitable for releasably holding the inner body part in place in a recess of the outer body part.

Typically, the inner body part is releasably held in place in the outer body part. The outer body part typically includes a recess for receiving the inner body part, in particular for assembly of the valve. In embodiments wherein the inner body part can be released from the outer body part, advantageously only the inner body part can be replaced for the purposes of maintenance and to mitigate any wear, corrosion, or erosion caused by the process fluid in the chamber.

Hence, preferably the outer body part has a recess for receiving the inner body part, more in particular for receiving and releasably holding the inner body part. In such embodiment, the inner body part can move out of the outer body part, e.g. slide out of the outer body part.

The valve body may comprise a crevice between the inner body part and outer body part and may also comprise a sealing element for said crevice.

Preferably, the outer body part is made of a first material and the inner body part is made of a second material, wherein the first material and second material are different from each other. This advantageously may allow for lower manufacturing costs.

The inner body part comprises a surface exposed to said chamber, i.e. providing a boundary of said chamber, and directly in contact with said fluid in the chamber in operation. Preferably, the inner body part is in part exposed to said chamber and is in part in direct contact with the fluid in operation.

The outer body part is for instance, without restriction, made of carbon steel, duplex stainless steel or austenitic stainless steel. In an interesting embodiment, the outer body part is made of carbon steel. Other materials are also possible, such as duplex stainless steel. However, the outer body part is in some embodiments made of material other than a duplex stainless steel. Use of a material other than a duplex stainless steel may be advantageous, firstly in view of the high content of expensive alloying elements in highly corrosion resistant duplex stainless steel and secondly, optionally, because of the need to control microstructure of duplex steel which may be challenging for larger equipment parts.

The outer body part is for instance made of carbon steel, austenitic stainless steel, and/or of stainless steel containing max. 27.0 wt.% Cr, e.g. containing 10.0 - 27.0 wt.% Cr, for instance stainless steel containing 10.0 - 25.0 wt.% Cr or e.g. containing 17.0 - 25.0 wt.% Cr.

The outer body part has for instance a wall thickness of at least 10 mm or at least 20 mm or at least 100 mm or at least 300 mm. Thereby the outer body part typically contributes to the mechanical strength of the valve.

The valve is configured to ensure that the outer body part is not in contact with the corrosive process fluid in operation.

The outer body part is for instance a single metal piece.

The outer body part comprises for instance a recess for holding the inner body part and at least two openings providing a passageway for fluid. Typically, the valve comprises a hollow element extending through each of said openings, such that fluid can flow to and from the chamber through said openings without direct contact with the outer body part. Typically, the outer body part also comprises an opening through which the stem of the flow regulating member extends. For example, the outer body part comprises an opening through which a part of the inner body part extends, wherein said part of the inner body part comprises the stem packing. Preferably, the valve body comprises two separate passageways or ports for fluid from and to the chamber, wherein said passageways both extend through the inner body part and the outer body part.

The inner body part is in operation in direct contact with the process fluid. The inner body part comprises a chamber and has a surface directly exposed to said chamber; said chamber receives the process fluid in operation. The chamber has for instance a concave and curved inner surface. Preferably, the parts of the inner body part providing the wall of the chamber, are entirely fitted in a recess of the outer body part in the assembled valve body.

The inner body part is for instance made of a corrosion resistant material, for example a duplex stainless steel.

The inner body part is for instance a sleeve that fits in the outer body part and for example fits through an opening in the outer body part. The outer body part for instance comprises a recess for receiving the inner body part. Preferably in this embodiment the inner body part is releasably held in place in the outer body part.

The inner body part is for instance provided as one part, as two parts, or for example as three or more parts, and is preferably a sleeve provided in one or more parts. Optionally, the inner body part is a single unitary part. Optionally the inner body part is a monolithic part.

Providing the inner body part is a single unitary part, made of duplex stainless steel, avoids the risk of crevice corrosion, compared to inner body parts made of two or more pieces. Moreover, in this embodiment the crevice between the inner and outer body part is less exposed to process fluid compared to embodiments with an inner body part provided as two or more parts, because the crevice between the inner body part can be completely shielded by the single unitary inner body part compared to an inner body part provided as two or more parts having crevices between them which crevices provide access for the fluid in the chamber to the outer body part. This is especially advantageous if the outer body part is made of carbon steel and susceptible to corrosion by the fluid in the chamber.

The inner body part and outer body part are preferably fit together. The inner body part and outer body part are for example connected to each other, for instance by mechanical connecting elements, such as pins, or bolting components. The inner body part is held in place in the outer body part. The inner body part and outer body part can also be mechanically joined by interference fit. For example, the seat connection piece may lock the inner body part in place. It may be observed that the mentioned connection means are suitable for releasably connecting the inner and outer body parts.

The inner body part and outer body part together form the valve body. The inner body part comprises the chamber and comprises at least two openings for fluid and typically also an opening for the stem.

The valve optionally comprises an O-ring and/or a sealing element between the inner body part and outer body part, for example to prevent external fluids to enter the crevice between the inner and outer body part.

The valve typically comprises for instance a sealing element between the inner body part and the valve seat. The sealing element may also be arranged simultaneously between the protective element and the inner body part.

In embodiments wherein the inner body part consists of two or more parts, the valve optionally comprises for instance an O-ring and/or a sealing element between the parts of the inner body part. The one or more sealing elements are e.g. a sealing ring or a metal-to-metal sealing. The sealing element is made e.g. of PTFE, PEEK, or is e.g. a metal sealing. Other types of non-metal sealings are also possible.

The valve for example also comprises a leak detection element. The leak detection element is optional and is a provision for detecting any leakage of fluid, in particular process fluid, from the inner body part towards the outer body part.

The valve seat is preferably releasably joined to the valve body. The valve seat preferably can be replaced for maintenance. The valve seat is preferably mechanically and releasably joined to the inner body part. In an example embodiment, the valve seat is supported by the inner body part. The valve seat is for example in direct contact with the inner body part. The valve seat and the inner body part are preferably separate equipment pieces; preferably disjoint parts. This provides the advantage that the valve seat can be replaced independently.

The valve seat is for example made of duplex stainless steel, with the same preferred alloys as for the internal body part. The valve seat is typically configured for contact with the flow regulating member, for example the plug, when the valve is in a closed position for sealing off the valve.

The valve seat optionally includes a notch prolongation. This prolongation advantageously contributes to the proper fitting with the plug. The valve seat for example includes a prolongation with a mating surface for engaging with the plug.

The valve for example comprises a seat connection piece. The seat connection piece is for example configured for holding the valve seat in place in the valve body. The seat connection piece is, for example, also configured for attachment of a tube or a pipe to the valve. The seat connection piece is for example attached or joined to the outer body part by preferably releasable connecting means, such as stud bolts; e.g. detachable connecting means. The inner body part is preferably in direct contact with the seat connection piece, for instance with a threaded flange. The seat connection piece can be removed from the valve body to allow for replacement of the valve seat and/or the flow regulating member, such as the stem with plug. The seat connection piece is for example made of duplex stainless steel. The same materials are preferred as for the inner body part. The seat connection piece is for instance a tubular piece having one end provided with means for connecting with a pipe, such as a flange, and at the other end fitted in an opening of the inner body part and in direct contact with the valve seat. The seat connection piece is for example configured for holding the inner body part in place in a recess of the outer body part. The seat connection piece is typically configured for holding the valve seat in place in the valve body. For example, the inner body part and the seat connection piece together provide a recess in which the valve seat fits, thereby holding the valve seat in place. The seat connection piece thereby is typically a pressure bearing part.

Removing the seat connection piece from the valve body may allow for removing the inner body part from the outer body part, in embodiments wherein the inner body part is releasably held in the outer body part. This can be used e.g. for replacement or inspection of the inner body part.

The valve in some embodiments comprises, as flow regulating member, a regulating stem and/or a stem and a plug. The regulating stem may comprise a stem and a plug. The plug can be moved through the chamber by the stem.

The valve comprises for example a regulating stem or a stem and a plug arranged for moving in the chamber between at least an opened position and a closed position. The plug is for example in contact with the valve seat in the closed position. The stem at one end extends through an opening in the wall of the valve. The stem preferably is provided with the plug at one end of the stem and at the other end the stem extends through an opening in the wall of the valve. For example, the stem and the plug are a unitary piece, or for example the stem and the plug are welded together. The stem and the plug are preferably both made of duplex stainless steel or other corrosion resistant stainless steels.

Optionally, the stem extends through an opening provided only in the inner body part, wherein preferably said opening is provided with a stem packing. Optionally, the inner body part comprises a surface that is exposed on the outside of the valve and that comprises an opening through which the stem extends. This embodiment provides an example of embodiment wherein a part but not all of the outer surface of the inner body part is covered by the outer body part.

The stem and the plug are in operation in contact with process fluid and are preferably made of highly corrosion resistant steel, such as duplex stainless steel, with the same preferred materials as for the inner body part.

In embodiments wherein the valve comprises an opening for the stem, the valve typically comprises a stem packing to provide for sealing. For example, a self-energizing stem packing is used.

Example materials for a self-energizing stem packing include: PTFE, PEEK, and a spring. The self-energizing stem packing comprises for instance a soft sealing, a support ring, and a spring. The spring is preferably made of stainless steel or other corrosion resistant materials.

The stem packing for instance comprises bushings and seals.

Energizing is advantageous for the seal functionality. Compared to a standard sealing used, a pre-compression and a regular check of this pre-compression is not required with a self-energizing stem packing. This means that the self-energizing stem packing requires no or less periodical maintenance and minimizes the risk of leakage during valve operation.

In a preferred embodiment, the valve comprises a first opening in the inner body part and a second opening in the outer body part. The first opening and the second opening are aligned with each other so as to allow fluid to flow between the second opening and the chamber. The opening in the inner body part and the opening in the outer body part together constitute an opening in the valve body.

Preferably, the valve comprises a protective element arranged inside the second opening so as to cover the outer body part from exposure to fluid in the second opening. The protective element can also be referred to as a pipe connection piece. The protective element is for instance tubular element, preferably having a radially extending end. The tubular element is inserted through the opening in the outer body part and preferably also through the opening in the inner body part aligned with said opening in the outer body part. The preferred radially extending end of the tubular element can be used for releasably attaching the tubular element to the outer body part. The preferred radially extending end is for instance provided at the outside of the valve body. An end of the tubular element, for example the radially extending end, which is arranged at the outside of the valve body, may also provide a flange for a pipe.

The protective element is preferably releasably, e.g. detachably, attached or joined to the valve body, in particular to the outer body part. Thereby the protective element may permit for easy replacement. Removing the protective element or pipe connection piece may also permit for release of the inner body part from the outer body part.

The protective element is for instance made of duplex stainless steel, with the same preferred alloys as for the inner body part.

The valve comprises connecting means for a pipe to connect to the valve, such as for example, screws. The protective element comprises an opening for fluid flow between the pipe and the valve chamber.

The valve may comprise a sealing system for sealing the protective element to the inner body part, for instance a seal ring. The sealing ring can be e.g. a soft sealing, such as a PTFE seal ring, or a metal to metal sealing.

The protective element is e.g. screwed to the outer body part. The valve for instance comprises a locking element holding the protective element in place and being attached or joined to the outer body part, e.g. with screws.

The inner body part is for example, without restriction, made from a bar by hot working and/or cold working and optional additional heat treatment. Hot working is a process were steel is plastically deformed above its recrystallization temperature so that new strain-free grains are formed due to the combined action of load and heat. Examples of hot working processes are forging, rolling and extrusion. Cold working refers to plastically deforming a steel below the recrystallization temperature. This causes a permanent change in the steel by introducing dislocations within its structure. The steel is then work-hardened. Some methods of cold working include cold rolling, cold pilgering and cold drawing. Steels are typically heat-treated to produce a great variety of microstructures and properties. Annealing, normalizing, tempering and quenching are different types of well-known heat treatments.

Heat treatment can be used to obtain the desired mechanical properties of the inner body part as well as desired corrosion resistance properties. The relatively smaller size, in particular wall thickness (mid-wall distance), of the inner body part, compared to a unitary valve body (single valve body) for an equivalent valve, contributes to achieving proper mechanical and corrosion resistance properties by heat treatment. This applies in particular in embodiments wherein the inner body part is made of duplex stainless steel. Heat treatment of a duplex stainless steel inner body part can also be performed on a bar element with pre-drilled hole. Also, for such a manufacture method, the relatively small size (mid-wall distance) of the inner body part contributes to achieving proper mechanical and corrosion resistance properties by heat treatment.

An aspect of the invention also pertains to such a method of making the inner body part. For example, there is provided a method of making an inner body part, of duplex stainless steel, comprising shaping a steel object to a shaped object, heat treatment, and optional further shaping; wherein the shaped object prior to heat treatment for instance comprises a drilled hole.

Suitable stainless steel for the inner body part of the valve, and for all other components of the valve that are in contact with the fluid in operation (e.g. the stem, the plug, the valve seat, the protective element) are duplex stainless steel.

The term 'typically' as used herein indicates features that are frequently used, but are not mandatory.

The term 'duplex stainless steel' refers to austenitic ferritic duplex stainless steel.

The term 'fluid' is used to include gas and liquid, as well as two-phase fluid flow. A fluid stream may comprise gas and/or liquid. A fluid stream can also be e.g. a suspension.

The inner body part of the valve, and for example all other components that are in contact with process fluid in operation (e.g. the stem, the plug, the valve seat, the protective element) are made of duplex stainless steel, suitable duplex stainless steel includes for example the steel available as Safurex^{®} steel and having composition 29Cr-6.5Ni-2Mo-N, which steel is also designated by ASME Code 2295-3 and by UNS S32906, or e.g. the steel available as DP28W^{™} steel and having composition 27Cr-7.6Ni-1 Mo-2.3W-N, which is also designated by ASME Code 2496-1 and by UNS S32808. The duplex steel for these components has for instance the composition (% by mass): C: max. 0.05, Si: max. 0.8, Mn: 0.3 - 4.0, Cr: 28 - 35, Ni: 3 - 10, Mo: 1.0 - 4.0, N: 0.2 - 0.6, Cu: max. 1.0 W: max. 2.0 S: max. 0.01 Ce: 0 - 0.2, balance Fe and (unavoidable) impurities. Preferably, the ferrite content is 30-70% by volume and more preferably 30-55%. More preferably, the steel contains (% by weight): C max. 0.02, max. 0.5 Si, Cr 29 to 33, Mo 1.0 to 2.0, N 0.36 to 0.55, Mn 0.3 to 1.0, balance Fe and impurities. Also suitable is a duplex stainless steel having the composition weight% (wt.%): C max 0.030; Si max 0.8; Mn max 2.0; Cr 29.0 to 31.0; Ni 5.0 to 9.0; Mo less than 4.0; W less than 4.0; N 0.25-0.45; Cu max 2.0; S max 0.02; P max 0.03; balance Fe and unavoidable occurring impurities; and wherein the content of Mo+W is greater than 3.0 but less than 5.0 or less than 4.0 (wt.%), furthermore preferably with a steel composition as described in US20180195158A1 hereby incorporated by reference. Also suitable is the duplex stainless steel having the composition: C max. 0.03: Mn 0.80 - 1.50: P max. 0.03: S max. 0.03: Si max. 0.80: Ni 5.8 - 7.5; Cr 28.0 - 30.0: Mo 1.50 - 2.60: N 0.30 - 0.40: Cu max. 0.80; balance Fe.

Other urea grade duplex stainless steels can also be used for the inner body part of the valve, and for all other components that are in contact with process fluid in operation (e.g. the stem, the plug, the valve seat, the protective element).

As used herein "highly corrosion resistant steel" in particular refers to urea grade steel, more in particular to urea grade duplex steel.

In an embodiment, the inner body part is made of duplex stainless steel; and preferably the outer body part is made of carbon steel. Preferably in these embodiments, the stem, the plug, the valve seat, the protective element and the inner body part are made of duplex stainless steel.

As a preferred feature, the inner body part is a sleeve that fits in the outer body part and fits through an opening in the outer body part and wherein the outer body part comprises a recess for receiving the inner body part and for releasably holding the inner body part in the outer body part. As a preferred feature, the outer body part is a single metal piece and wherein the inner body part is a single unitary part.

In conclusion, the invention pertains to a plant and/or urea production process in a plant comprising a valve body comprising an inner body part and an outer body part and the method of modifying an existing urea production plant.

The term 'plant' as used herein indicates an installation and can also be referred to as system. The term 'carbamate' refers to ammonium carbamate, as that term is used in the art of urea production.

As used herein, HP (high pressure) indicates a pressure of e.g. at least 100 bara, e.g. 120-250 bara, or e.g. 110-160 bara, MP (medium pressure) indicates a pressure of e.g. 15-80 bara, 20-80 bara, and LP (low pressure) indicates a pressure of e.g. 1-10 bara, e.g. 4-10 bara; these pressure ranges are for process streams of the urea plant and not necessarily the same for steam and heating fluids. The abbreviation "bara" means bar absolute. Pressures are absolute pressures unless indicated otherwise.

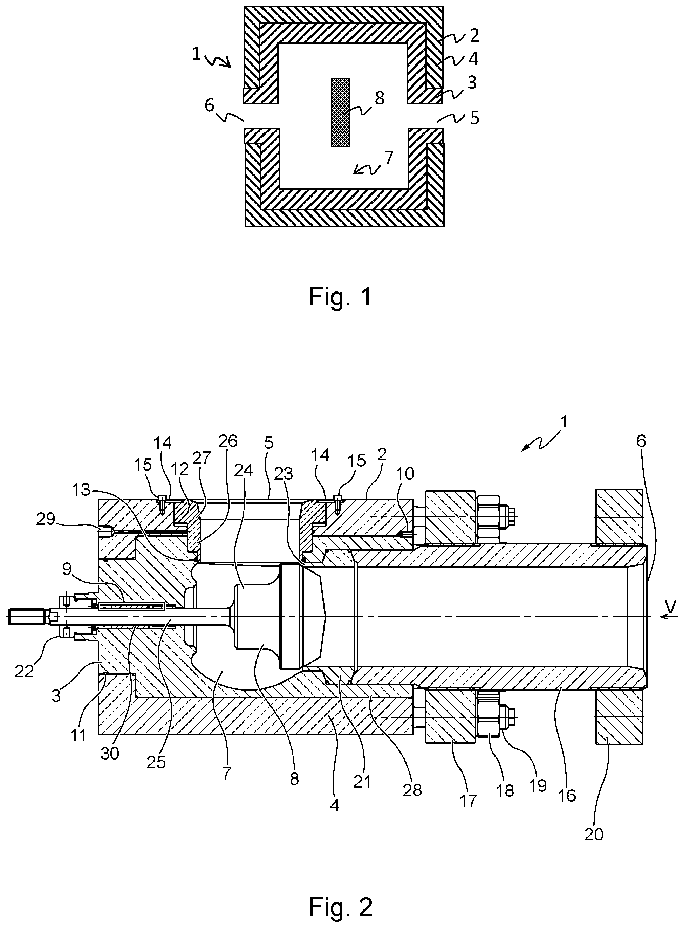

**Fig. 1** schematically shows an example valve (1) according to the invention and/or as used in the invention. The valve (1) comprises a valve body (2) with an inner body part (3) and an outer body part (4) according to the invention. The valve (1) further comprises a first opening (5) and a second opening (6) for fluid to enter and exit the valve body (2), in particular to enter and exit a chamber (7) for holding fluid of the valve body (2). The valve (1) also comprises a flow regulating member (8), for example a regulating stem or a disc. The flow regulating member (8) can move between at least two different positions for regulating the fluid flow. The flow regulating member (8) is at least in part located in the chamber (7). The flow regulating member (8) may in part extend through an opening of the valve body (2).

**Fig. 2** shows an example valve (1) according to the invention and/or as used in the invention. The valve (1) comprises a valve body (2) which comprises an inner body part (3) and an outer body part (4). The inner body part (3) is located in a recess (28) of the outer body part (4). The inner body part (3) and an outer body part (4) as illustrated are e.g. both single unitary pieces, e.g. of two different types of steel. The recess (28) and the outer surface of the inner body part (3) have a mating shape, as illustrated, without gaps between them.

The valve (1) further comprises a first opening (5) and a second opening (6) for fluid to enter and exit the valve body (2), in particular to enter and exit a chamber (7) for holding fluid of the valve body (2). The flow regulating member (8) is provided as a plug (24) on a stem (25). A stem packing (9) is provided between the stem (25) and the inner body part (3). The stem (25) extends through an opening (30) in the inner body part (3). The stem (25) can move in the chamber (7) for regulating the fluid flow.

The inner (3) and outer (4) body parts are held in place using optional pins (10).

An O-ring (11) is located between the inner body part (3) and the outer body part (4), for example to provide a sealing against external fluids.

An opening (5) for fluid flow is provided through both the inner and outer body part (26, 27). A pipe connection piece (12) provides an example protective element arranged in the opening (27) in the outer body part (4) and, optionally, also in the opening (26) in the inner body part (3). Thereby the outer body part (4) is protected against the corrosive fluid in the opening (5). A seal ring (13) is provided between the pipe connection piece (12) and the inner body part (3). The pipe connection piece (12) is connected, at the radially extending end thereof, to the outer body part (4) using the optional plate (14) and screws (15). A pipe can be connected to the outer end of the pipe connection piece (12). The openings (26,27) in the inner respectively outer body part (3, 4) are aligned with each other so as to provide the opening (5) for fluid flow through the valve body (2).

A seat connection piece (16) is provided for holding the inner body part (3) in place in the outer body part (4). The seat connection piece (16) is attached or joined to the valve outer body part (4) using a seat flange (17) and bolts and nuts (18,19). The seat connection piece (16) comprises a flange (20) for connection with a pipe.

In an example embodiment, the seat connection piece (16) and seat flange (17) can be detached and the inner body part (3), being a unitary metal piece, can exit the recess (28) of the outer body part (4) through the opening in the outer body part at the side with seat flange (17) (right hand side in the figure) after removal of the pipe connection piece (12), in example embodiments wherein the pipe connection piece (12) protrudes in the opening (26) in the inner body part, and after suitable detachment of the stem (25).

The valve (1) comprises a valve seat (21) configured for sealing contact with the regulating stem, in particular for sealing contact with a plug. The valve seat (21) includes a notch prolongation (23) for engaging with the plug.

The regulating stem is, in this example, at one end attached to an actuator, using e.g. the screw (22). Alternatively, the valve (1) can be manually operated.

The valve (1) also comprises a leak detection element (29). The leak detection element (29) is optional and is a provision for detecting any leakage of fluid, in particular process fluid, from the inner body part (3) towards the outer body part (4).

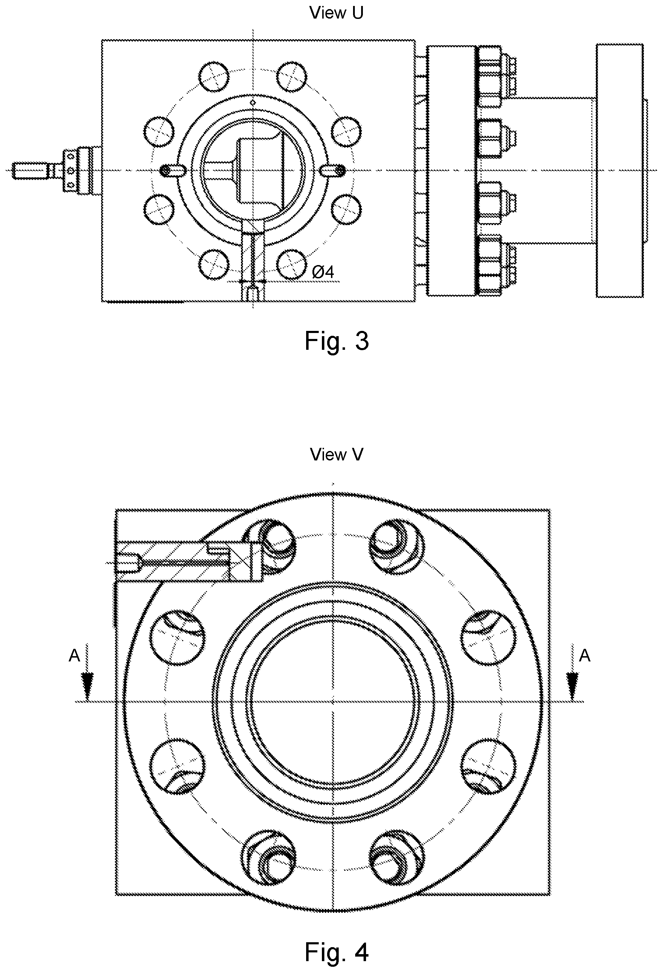

**Fig. 3** shows the view U of the valve (1) of Fig. 2.

**Fig. 4** shows the view V of the valve (1) of Fig. 2.

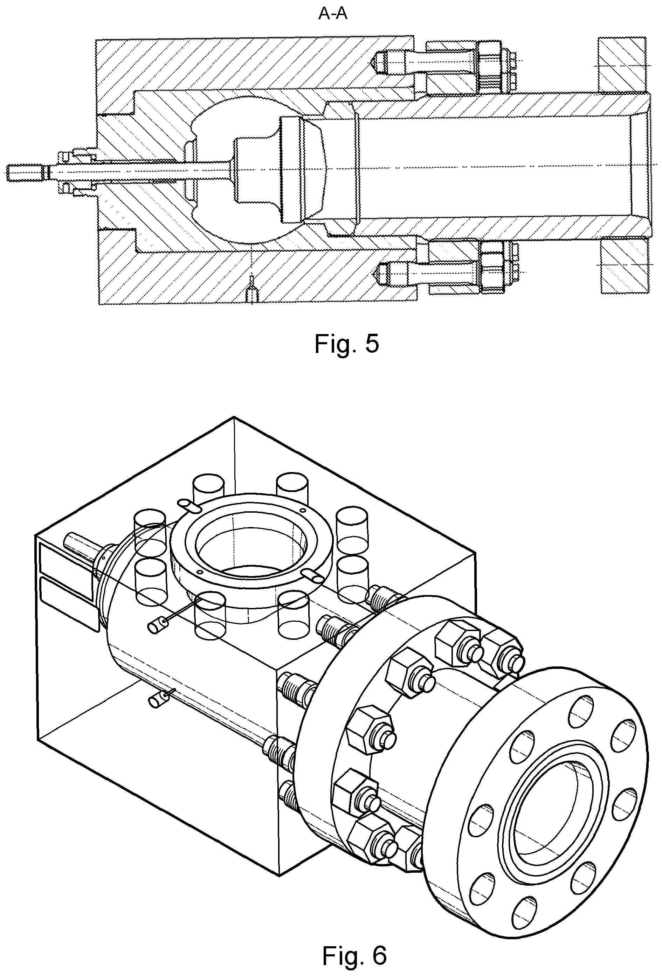

**Fig. 5** shows the view along A-A (see Fig. 4) of the valve (1) of Fig. 2.

**Fig. 6** shows an isometric view of the valve (1) of Fig. 2.

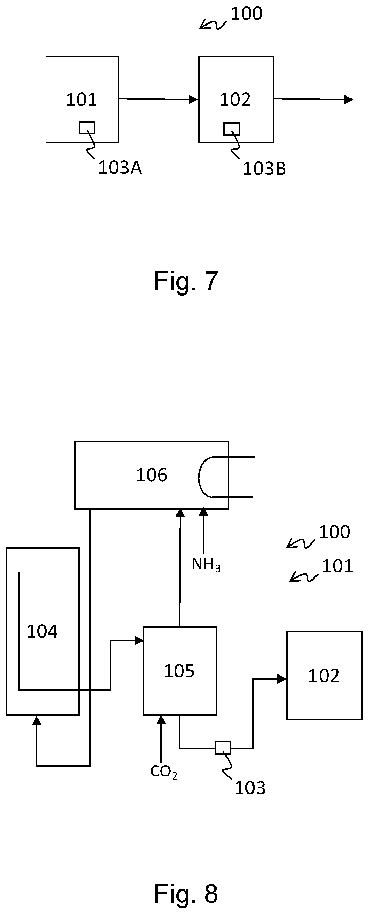

**Fig. 7** schematically shows an example urea plant (100) according to the invention. The urea plant (100) comprises a synthesis section (101) and a recovery section (102) receiving urea solution from the synthesis section (101) and having an outlet for purified urea solution. The urea plant comprises the valve (103A;103B) comprising an inner and an outer body part in the synthesis section (101) and/or in the recovery section (102).

**Fig. 8** schematically shows an example high pressure (HP) synthesis section of an example urea production plant (100) according to the invention. The HP synthesis section (101) comprises a reaction zone (104) comprising a reactor (104). The HP synthesis section (101) also comprises a high pressure (HP) stripper (105) and a high pressure (HP)carbamate condensation zone (106) comprising a carbamate condenser (106). Particularly, the HP stripper (105) is illustrated as a stripper of the CO₂ stripping type, having an inlet for CO₂ feed at the bottom and an outlet for stripped urea solution at the bottom connected to the recovery section (102). The HP carbamate condenser (106) is illustrated as a horizontal condenser with gas to be condensed from the stripper (105) in the shell, also receiving NH₃ feed, and with cooling fluid in a U-shaped tube bundle. Carbamate solution from the HP carbamate condenser (106) is supplied to a bottom inlet of the reactor (104). Merely as an example, the flow line for stripped urea solution from the HP stripper (105) to the recovery section (102) may comprise the valve (103) according to the invention, preferably for reducing the pressure of the stripped urea solution from high pressure (HP) to medium pressure (MP) or to low pressure (LP).

## Claims

1. A urea production plant (100) comprising a high pressure (HP) synthesis section (101) comprising a reaction zone (104) and a valve (1, 103), wherein the valve comprises a valve body (2), an inlet for fluid (5), an outlet for fluid (6), a flow-regulating member (8), a chamber (7) for receiving corrosive process fluid in the valve body (2), and a valve seat (21), wherein the valve body (2) comprises an outer body part (4) and an inner body part (3) fit together and/or mechanically joined to each other,
whereby the outer body part (4) is made of a first material and whereby the inner body part (3) is made of a second material, wherein the first material and the second material are different from each other, wherein the second material is duplex stainless steel; wherein the inner body part (3) comprises a surface exposed to said chamber (7) and directly in contact with said corrosive process fluid in the chamber (7) in operation, wherein the valve is configured to ensure that the outer body part is not in contact with the corrosive process fluid in operation.

2. The plant according to claim 1, wherein the HP synthesis section (101) comprises a HP stripper and a HP carbamate condensation zone (106).

3. The plant according to claim 1 or 2, wherein the inner body part is a single unitary part.

4. The plant according to any of the preceding claims, wherein the flow-regulating member (8) comprises a regulating stem or a stem (25) and a plug (24) arranged for moving in the chamber (7) between at least two different positions for regulating fluid flow; and wherein the stem (25) extends through an opening (30) of the inner body part (3) provided with a stem packing (9).

5. The plant according to any of the preceding claims, wherein the valve seat (21) is supported by and in direct contact with the inner body part (3).

6. The plant according to any of the preceding claims, wherein the valve (1, 103) comprises an opening (26) in the inner body part and an opening (27) in the outer body part (4), said openings (26, 27) being aligned with each other so as to allow the corrosive process fluid flow between the opening (26) in the inner body part (3) and the chamber (7); wherein the valve (1, 103) comprises a protective element (12) arranged inside the opening (27) in the outer body part (4) so as to cover the outer body part (4) from exposure to the corrosive process fluid in the opening (27) in the outer body part (4), wherein the protective element (12) optionally also extends through the opening (26) in the inner body (3).

7. The plant according to claim 6, wherein the protective element (12) is a tubular element inserted in both the opening (26) in the inner body part (3) and the opening (27) in the outer body part (4).

8. The plant according to any of the preceding claims, wherein the valve (1, 103) comprises a seat connection piece (16) which is attached to the outer body part (4) by releasable connecting means and which holds the inner body part (3) in place in a recess (28) of the outer body part (4); wherein preferably the seat connection piece (16) also holds the valve seat (21) in place in the valve body (2).

9. The plant according to any of the preceding claims, wherein the outer body part includes a recess for receiving the inner body part, and wherein the inner body part is releasably held in place in the outer body part.

10. The plant according to any of the preceding claims, wherein the inner body part is a sleeve that fits in a recess of the outer body part and fits through an opening in the outer body part.

11. The plant according to any of the preceding claims, wherein the inner body part is mechanically held in a recess of the outer body part with an interference fit or a friction fit.

12. Use of a valve in a carbamate environment, wherein the valve comprises a valve body (2), an inlet for fluid (5), an outlet for fluid (6), a flow-regulating member (8), a chamber (7) for receiving fluid in the valve body (2), and a valve seat (21), wherein the valve body (2) comprises an outer body part (4) and an inner body part (3) fit together and/or mechanically joined to each other, whereby the outer body part (4) is made of a first material and whereby the inner body part (3) is made of a second material that is duplex stainless steel, wherein the first material and the second material are different from each other; wherein the inner body part (3) comprises a surface exposed to said chamber (7) and directly in contact with said fluid in the chamber (7) in operation,
said use comprising exposing the inner body part (3) at high pressure of at least 100 bara to a liquid comprising carbamate and/or to a gas stream comprising CO₂ and NH₃, wherein the valve is configured to ensure that the outer body part is not in contact with corrosive process fluid in operation.

13. Use according to claim 12, wherein said exposure is at high pressure in the range 120-250 bara.

14. The use according to claim 12 or 13, wherein the valve has the features as defined in any of claims 3 -11.

15. A urea production process comprising forming urea from NH₃ and CO₂ in a plant according to any of claims 1-11.

16. A urea production process according to claim 15, wherein the process is of the CO₂ stripping, the NH₃ stripping, or the self-stripping type.

17. A method of modifying an existing urea production plant comprising a first valve, by replacing the first valve by a replacement valve wherein the replacement valve comprises a valve body (2), an inlet for fluid (5), an outlet for fluid (6), a flow-regulating member (8), a chamber (7) for receiving corrosive process fluid in the valve body (2), and a valve seat (21), wherein the valve body (2) comprises an outer body part (4) and an inner body part (3) fit together and/or mechanically joined to each other, whereby the outer body part (4) is made of a first material and whereby the inner body part (3) is made of a second material that is duplex stainless steel, wherein the first material and the second material are different from each other; wherein the inner body part (3) comprises a surface exposed to said chamber (7) and directly in contact with said fluid in the chamber (7) in operation; wherein the replacement valve is configured to ensure that the outer body part is not in contact with the corrosive fluid in operation; and wherein the replacement valve (1, 103) is comprised in a high pressure (HP) synthesis section (101) of the urea production plant (100), wherein the HP synthesis section (101) comprises a reaction zone (104).

## Patentansprüche

1. Harnstoffherstellungsanlage (100), umfassend einen Hochdruck-Syntheseabschnitt (HD-Syntheseabschnitt) (101), umfassend eine Reaktionszone (104) und ein Ventil (1, 103), wobei das Ventil einen Ventilkörper (2), einen Einlass für Fluid (5), einen Auslass für Fluid (6) ein Strömungsregulierungselement (8), eine Kammer (7) zum Aufnehmen von korrosivem Prozessfluid in den Ventilkörper (2) und einen Ventilsitz (21) umfasst, wobei der Ventilkörper (2) ein äußeres Körperteil (4) und ein inneres Körperteil (3) umfasst, die zusammenpassen und/oder mechanisch miteinander verbunden sind,
wodurch das äußere Körperteil (4) aus einem ersten Material hergestellt ist, und wodurch das innere Körperteil (3) aus einem zweiten Material hergestellt ist, wobei sich das erste Material und das zweite Material voneinander unterscheiden, wobei das zweite Material rostfreier Duplex-Stahl ist; wobei das innere Körperteil (3) eine Oberfläche umfasst, die der Kammer (7) exponiert ist und bei Betrieb direkt in Kontakt mit dem korrosiven Prozessfluid in der Kammer (7) ist, wobei das Ventil dazu konfiguriert ist sicherzustellen, dass das äußere Körperteil bei Betrieb nicht mit dem korrosiven Prozessfluid in Kontakt steht.

2. Anlage nach Anspruch 1, wobei der HD-Syntheseabschnitt (101) einen HD-Stripper und eine HD-Carbamatkondensationszone (106) umfasst.

3. Anlage nach Anspruch 1 oder 2, wobei das innere Körperteil ein einziges unitäres Teil ist.

4. Anlage nach einem der vorstehenden Ansprüche, wobei das Strömungsregulierungselement (8) einen Regulierungsschaft oder einen Schaft (25) und einen Stopfen (24) umfasst, eingerichtet zum Bewegen der Kammer (7) zwischen mindestens zwei unterschiedlichen Positionen zum Regulieren des Fluidstroms; und wobei sich der Schaft (25) durch eine Öffnung (30) des inneren Körperteils (3), versehen mit einer Schaftdichtung (9), erstreckt.

5. Anlage nach einem der vorstehenden Ansprüche, wobei der Ventilsitz (21) durch und in direktem Kontakt mit dem inneren Körperteil (3) getragen wird.

6. Anlage nach einem der vorstehenden Ansprüche, wobei das Ventil (1, 103) eine Öffnung (26) in dem inneren Körperteil und eine Öffnung (27) in dem äußeren Körperteil (4) umfasst, wobei die Öffnungen (26, 27) zueinander derart ausgerichtet sind, dass sie es dem korrosiven Prozessfluid erlauben, zwischen der Öffnung (26) in dem inneren Körperteil (3) und der Kammer (7) zu strömen; wobei das Ventil (1, 103) ein Schutzelement (12) umfasst, eingerichtet innerhalb der Öffnung (27) in dem äußeren Körperteil (4), um das äußere Körperteil (4) gegen Exposition mit dem korrosiven Prozessfluid in der Öffnung (27) in dem äußeren Körperteil (4) zu bedecken, wobei sich das Schutzelement (12) optional durch die Öffnung (26) in dem Innenkörper (3) erstreckt.

7. Anlage nach Anspruch 6, wobei das Schutzelement (12) ein röhrenförmiges Element ist, das sowohl in die Öffnung (26) in dem inneren Körperteil (3) als auch in die Öffnung (27) in dem äußeren Körperteil (4) eingesetzt ist.

8. Anlage nach einem der vorstehenden Ansprüche, wobei das Ventil (1, 103) ein Sitzverbindungsstück (16) umfasst, das an dem äußeren Körperteil (4) durch lösbare Verbindungsmittel angebracht ist, und das das innere Körperteil (3) in einer Vertiefung (28) des äußeren Körperteils (4) an Ort und Stelle hält; wobei das Sitzverbindungsstück (16) bevorzugt auch den Ventilsitz (21) in dem Ventilkörper (2) an Ort und Stelle hält.

9. Anlage nach einem der vorstehenden Ansprüche, wobei das äußere Körperteil eine Vertiefung zum Aufnehmen des inneren Körperteils beinhaltet, und wobei das innere Körperteil lösbar in dem äußeren Körperteil an Ort und Stelle gehalten wird.

10. Anlage nach einem der vorstehenden Ansprüche, wobei das innere Körperteil eine Hülse ist, die in eine Vertiefung des äußeren Körperteils passt und durch eine Öffnung in das äußere Körperteil passt.

11. Anlage nach einem der vorstehenden Ansprüche, wobei das innere Körperteil mechanisch in einer Vertiefung des äußeren Körperteils mit einer Press- oder einer Reibpassung gehalten wird.

12. Verwendung eines Ventils in einer Carbamatumgebung, wobei das Ventil einen Ventilkörper (2), einen Einlass für Fluid (5), einen Auslass für Fluid (6), ein Flussregulierungselement (8), eine Kammer (7) zum Aufnehmen von Fluid in dem Ventilkörper (2) und einen Ventilsitz (21) umfasst, wobei der Ventilkörper (2) ein äußeres Körperteil (4) und ein inneres Körperteil (3) umfasst, die zusammenpassen und/oder mechanisch miteinander verbunden sind, wodurch das äußere Körperteil (4) aus einem ersten Material hergestellt ist, und wodurch das innere Körperteil (3) aus einem zweiten Material, das rostfreier Duplex-Stahl ist, hergestellt ist, wobei sich das erste Material und das zweite Material voneinander unterscheiden; wobei das innere Körperteil (3) eine Oberfläche umfasst, die mit der Kammer (7) exponiert ist und bei Betrieb direkt in Kontakt mit dem Fluid in der Kammer (7) steht,
wobei die Verwendung das Exponieren des inneren Körperteils (3) bei hohem Druck von mindestens 100 bara mit einer Flüssigkeit umfasst, die Carbamat umfasst, und/oder mit einem Gasstrom, umfassend CO₂ und NH₃, wobei das Ventil konfiguriert ist, um sicherzustellen, dass das äußere Körperteil bei Betrieb nicht mit korrosivem Prozessfluid in Kontakt steht.

13. Verwendung nach Anspruch 12, wobei die Exposition bei hohem Druck in dem Bereich von 120-250 bara ist.

14. Verwendung nach Anspruch 12 oder 13, wobei das Ventil die Merkmale wie in einem der Ansprüche 3 bis 11 definiert, hat.

15. Harnstoffherstellungsprozess, umfassend Bilden von Harnstoff aus NH₃ und CO₂ in einer Anlage nach einem der Ansprüche 1 bis 11.

16. Harnstoffherstellungsprozess nach Anspruch 15, wobei der Prozess vom Typ CO₂-Stripping, NH₃-Stripping oder Self-Stripping ist.

17. Verfahren zum Modifizieren einer bestehenden Harnstoffherstellungsanlage, umfassend ein erstes Ventil, durch Ersetzen des ersten Ventils durch ein Ersatzventil, wobei das Ersatzventil einen Ventilkörper (2), einen Einlass für Fluid (5), einen Auslass für Fluid (6), ein Strömungsregulierungselement (8), eine Kammer (7) zum Aufnehmen von korrosivem Prozessfluid in den Ventilkörper (2), und einen Ventilsitz (21) umfasst, wobei der Ventilkörper (2) ein äußeres Körperteil (4) und ein inneres Körperteil (3) umfasst, die zusammenpassen und/oder mechanisch miteinander verbunden sind, wodurch das äußere Körperteil (4) aus einem ersten Material hergestellt ist, und wodurch das innere Körperteil (3) aus einem zweiten Material hergestellt ist, das rostfreier Duplex-Strahl ist, wobei sich das erste Material und das zweite Material voneinander unterscheiden; wobei das innere Körperteil (3) eine Oberfläche umfasst, die mit der Kammer (7) exponiert ist und bei Betrieb direkt mit dem Fluid in der Kammer (7) in Kontakt steht; wobei das Ersatzventil konfiguriert ist, um sicherzustellen, dass das äußere Körperteil bei Betrieb nicht mit dem korrosiven Fluid in Kontakt steht; und wobei das Ersatzventil (1, 103) in einem Hochdruck-Syntheseabschnitt (HD-Syntheseabschnitt) (101) der Harnstoffherstellungsanlage (100) enthalten ist, wobei der HD-Syntheseabschnitt (101) eine Reaktionszone (104) umfasst.

## Revendications

1. Installation de production d'urée (100) comprenant une section de synthèse haute pression (HP) (101) comprenant une zone de réaction (104) et une valve (1, 103), dans laquelle la valve comprend un corps de valve (2), une entrée pour fluide (5), une sortie pour fluide (6), un élément de régulation de flux (8), une chambre (7) pour recevoir le fluide de traitement corrosif dans le corps de valve (2) et un siège de valve (21), dans laquelle le corps de valve (2) comprend une partie de corps externe (4) et une partie de corps interne (3) montées ensemble et/ou mécaniquement assemblées entre elles,
moyennant quoi la partie de corps externe (4) est réalisée avec un premier matériau et moyennant quoi la partie de corps interne (3) est réalisée avec un deuxième matériau, dans laquelle le premier matériau et le deuxième matériau sont différents l'un de l'autre, dans laquelle le deuxième matériau est de l'acier inoxydable duplex ; dans laquelle la partie de corps interne (3) comprend une surface exposée par rapport à ladite chambre (7) et directement en contact avec ledit fluide de traitement corrosif dans la chambre (7) en fonctionnement, dans laquelle la valve est configurée pour garantir que la partie de corps externe n'est pas en contact avec le fluide de traitement corrosif en fonctionnement.

2. Installation selon la revendication 1, dans laquelle la section de synthèse HP (101) comprend un extracteur HP et une zone de condensation de carbamate HP (106).

3. Installation selon la revendication 1 ou 2, dans laquelle la partie de corps interne est une partie unitaire unique.

4. Installation selon l'une quelconque des revendications précédentes, dans laquelle l'élément de régulation de flux (8) comprend une tige de régulation ou une tige (25) et un bouchon (24) agencés pour se déplacer dans la chambre (7) entre au moins deux positions différentes pour réguler le flux de fluide ; et dans laquelle la tige (25) s'étend à travers une ouverture (30) de la partie de corps interne (3) prévue avec une garniture de tige (9).

5. Installation selon l'une quelconque des revendications précédentes, dans laquelle le siège de valve (21) est supporté par et est en contact direct avec la partie de corps interne (3).

6. Installation selon l'une quelconque des revendications précédentes, dans laquelle la valve (1, 103) comprend une ouverture (26) dans la partie de corps interne et une ouverture (27) dans la partie de corps externe (4), lesdites ouvertures (26, 27) étant alignées les unes avec les autres afin de permettre au fluide de traitement corrosif de s'écouler entre l'ouverture (26) dans la partie de corps interne (3) et la chambre (7) ; dans laquelle la valve (1, 103) comprend un élément de protection (12) agencé à l'intérieur de l'ouverture (27) dans la partie de corps externe (4) afin de recouvrir la partie de corps externe (4) par rapport à l'exposition au fluide de traitement corrosif dans l'ouverture (27) dans la partie de corps externe (4), dans laquelle l'élément de protection (12) s'étend également facultativement à travers l'ouverture (26) dans le corps interne (3).

7. Installation selon la revendication 6, dans laquelle l'élément de protection (12) est un élément tubulaire inséré à la fois dans l'ouverture (26) dans la partie de corps interne (3) et l'ouverture (27) dans la partie de corps externe (4).

8. Installation selon l'une quelconque des revendications précédentes, dans laquelle la valve (1, 103) comprend une pièce de raccordement de siège (16) qui est fixée à la partie de corps externe (4) par des moyens de raccordement amovibles et qui maintient la partie de corps interne (3) en place dans un évidement (28) de la partie de corps externe (4) ; dans laquelle de préférence la pièce de raccordement de siège (16) maintient également le siège de valve (21) en place dans le corps de valve (2).

9. Installation selon l'une quelconque des revendications précédentes, dans laquelle la partie de corps externe comprend un évidement pour recevoir la partie de corps interne, et dans laquelle la partie de corps interne est maintenue, de manière amovible, en place dans la partie de corps externe.

10. Installation selon l'une quelconque des revendications précédentes, dans laquelle la partie de corps interne est un manchon qui se monte dans un évidement de la partie de corps externe et se monte à travers une ouverture dans la partie de corps externe.

11. Installation selon l'une quelconque des revendications précédentes, dans laquelle la partie de corps interne est mécaniquement maintenue dans un évidement de la partie de corps externe avec un ajustement avec serrage ou un ajustement avec friction.

12. Utilisation d'une valve dans un environnement de carbamate, dans laquelle la valve comprend un corps de valve (2), une entrée pour fluide (5), une sortie pour fluide (6), un élément de régulation de flux (8), une chambre (7) pour recevoir le fluide dans le corps de valve (2) et un siège de valve (21), dans laquelle le corps de valve (2) comprend une partie de corps externe (4) et une partie de corps interne (3) montées ensemble et/ou mécaniquement assemblées entre elles, moyennant quoi la partie de corps externe (4) est réalisée avec un premier matériau et moyennant quoi la partie de corps interne (3) est réalisée avec un deuxième matériau qui est un acier inoxydable duplex, dans laquelle le premier matériau et le deuxième matériau sont différents l'un de l'autre ; dans laquelle la partie de corps interne (3) comprend une surface exposée par rapport à ladite chambre (7) et directement en contact avec ledit fluide dans la chambre (7) en fonctionnement,
ladite utilisation comprenant l'exposition de la partie de corps interne (3) à haute pression d'au moins 100 baras à un liquide comprenant du carbamate et/ou un flux de gaz comprenant du CO₂ et du NH₃, dans laquelle la valve est configurée pour garantir que la partie de corps externe n'est pas en contact avec le fluide de traitement corrosif en fonctionnement.

13. Utilisation selon la revendication 12, dans laquelle ladite exposition est à haute pression dans la plage de 120-250 baras.

14. Utilisation selon la revendication 12 ou 13, dans laquelle la valve a la caractéristique, telle que définie selon l'une quelconque des revendications 3 à 11.

15. Procédé de production d'urée comprenant la formation d'urée à partir de NH₃ et de CO₂ dans une installation selon l'une quelconque des revendications 1 à 11.

16. Procédé de production d'urée selon la revendication 15, dans lequel le procédé est du type à extraction de CO₂, extraction de NH₃ ou à auto-extraction.

17. Procédé pour modifier une installation de production d'urée existante comprenant une première valve, en remplaçant la première valve par une valve de remplacement, dans lequel la valve de remplacement comprend un corps de valve (2), une entrée pour fluide (5), une sortie pour fluide (6), un élément de régulation de flux (8), une chambre (7) pour recevoir le fluide de traitement corrosif dans le corps de valve (2) et un siège de valve (21), dans lequel le corps de valve (2) comprend une partie de corps externe (4) et une partie de corps interne (3) montées ensemble et/ou mécaniquement assemblées entre elles, moyennant quoi la partie de corps externe (4) est réalisée avec un premier matériau et moyennant quoi la partie de corps interne (3) est réalisée avec un deuxième matériau qui est de l'acier inoxydable duplex, dans lequel le premier matériau et le deuxième matériau sont différents l'un de l'autre ; dans lequel la partie de corps interne (3) comprend une surface exposée à ladite chambre (7) et directement en contact avec ledit fluide dans la chambre (7) en fonctionnement ; dans lequel la valve de remplacement est configurée pour garantir que la partie de corps externe n'est pas en contact avec le fluide corrosif en fonctionnement ; et dans lequel la valve de remplacement (1, 103) est comprise dans une section de synthèse haute pression (HP) (101) de l'installation de production d'urée (100), dans lequel la section de synthèse HP (101) comprend une zone de réaction (104).
